Europäisches Patentamt

European Patent Office

Office européen des brevets

(19)

(11) Numéro de publication: **0 214 035 B1**

(12) **FASCICULE DE BREVET EUROPEEN**

(45) Date de publication de fascicule du brevet:
21.11.91

(51) Int. Cl.⁵: **C08H 1/06**, **A61L 27/00**, **A61L 15/16**, **G02B 1/04**

(21) Numéro de dépôt: 86401794.2

(22) Date de dépôt: **11.08.86**

(54) Nouvelles préparations de collagène placentaire, leur procédé d'extraction et leurs applications.

(30) Priorité: 02.09.85 FR 8513004

(43) Date de publication de la demande:
11.03.87 Bulletin 87/11

(45) Mention de la délivrance du brevet:
21.11.91 Bulletin 91/47

(84) Etats contractants désignés:
BE CH DE FR GB IT LI NL SE

(56) Documents cités:
EP-A- 0 080 956
DE-A- 2 462 222
DE-A- 2 616 939
US-A- 4 268 131
US-A- 4 505 855

DIE ANGEWANDTE MAKROMOLEKULARE
CHEMIE, vol. 111, no. 1701, janvier 1983, pages 107-122, Hüthig & Wepf Verlag, Heidelberg, DE; M.Z. ABEDIN et al.: "Collagen heterogeneity and its functional significance"

(73) Titulaire: **PASTEUR MERIEUX SERUMS ET VACCINS**
58, avenue Leclerc
F-69007 Lyon(FR)

(72) Inventeur: **Tayot, Jean-Louis**
**1 rue des Greffières**
**F-69890 La Tour de Salvagny(FR)**
Inventeur: **Tardy, Michel**
**165bis rue Joliot Curie**
**F-69005 Lyon(FR)**

(74) Mandataire: **Lemoine, Michel et al**
**Cabinet Michel Lemoine et Bernasconi 13**
**Boulevard des Batignolles**
**F-75008 Paris(FR)**

DIE ANGEWANDTE MAKROMOLEKULARE
CHEMIE, vol. 82, no. 1276, 1979, pages
171-186, Hüthig & Wepf Verlag, Berlin, DE; R.
RIEMSCHNEIDER et al.: "Pepsin-solubilized
collagen from cow placenta"

BIOCHEMISTRY, vol. 18, no. 14, 1979, pages
3089-3097, American Chemical Society; T.F.
KRESINA et al.: "Isolation and characterization of basement membrane collagen from
human placental tissue. Evidence for the
presence ot two genetically distinct collagen chains"

## Description

La présente invention a trait à la préparation d'un gel ou d'une solution physiologique de collagène, non modifié, transparent et hémocompatible à partir de placenta.

De façon plus générale, elle concerne un procédé d'extraction de collagènes de placenta humain ou animal, permettant d'isoler un collagène enrichi en type IV et exploitable industriellement.

Elle se rapporte plus précisément à un procédé pour préparer des solutions ou des gels de collagène acides ou neutres, parfaitement transparents sur une grande épaisseur.

Depuis un certain nombre d'années, on a cherché à mettre au point des gels de collagène transparents pour la fabrication de lentilles de contact. Plus récemment, leur utilisation a été recherchée pour greffer ou insérer ces produits dans la cornée elle-même, voire même dans l'oeil. On comprend l'importance de disposer de collagène identique au, ou le plus proche possible du, collagène humain pour une utilisation chirurgicale dans l'espèce humaine si l'on veut éviter toute réaction inflammatoire ou immunologique. Or, aujourd'hui, les seules préparations de collagène disponibles commercialement en quantité suffisante et à un prix acceptable sont obtenues à partir de queues ou tendons de rat ou à partir de peaux de jeunes animaux (veau par exemple). Elles consistent presque uniquement en collagène de type I. Les autres types II, III, IV, V et autres sont uniquement préparés par des chercheurs spécialisés selon des méthodes très complexes et à des prix trop élevés pour permettre une exploitation à grande échelle.

Il a été affirmé dans de nombreuses publications que les molécules de collagène animal sont peu ou pas antigéniques lorsqu'elles ont été obtenues par un procédé utilisant la pepsine qui élimine les peptides terminaux (télopeptides) non hélicoïdaux. La structure en triple hélice du collagène, résistante à l'action de la pepsine, étant voisine dans toutes espèces animales; serait peu antigénique. Or, il semble que ce caractère non antigénique du collagène ayant été soumis à l'action de la pepsine doit être remis en cause. Il est, en effet, connu maintenant que ces structures sont antigéniques dans une autre espèce, ceci ayant permis de préparer des antisérums spécifiques de tel ou tel type de collagène bovin ou humain, chez le lapin par exemple. Il faut, en outre, tenir compte de la présence toujours possible d'impuretés protéiques animales antigéniques dans de telles préparations.

Depuis quelques années, l'injection de collagène I d'origine bovine a été pratiquée de manière intensive dans l'espèce humaine, pour combler les cicatrices ou dans des techniques de chirurgie réparatrice. Ces essais ont permis de mettre en évidence un certain nombre d'inconvénients dont deux sont importants :
- l'apparition de réactions immunologiques chez 1 à 3 % des sujets ;
- l'apparition de réactions de coagulation du sang localement lorsque le produit est injecté malencontreusement dans un vaisseau ou un capillaire sanguin.

Ces faits soulignent donc toute l'importance qu'il y aurait à obtenir des gels de collagène humain qui soient non seulement transparents pour être utilisés en ophtalmologie dans l'espèce humaine sans risque de réactions immunologiques indésirables, mais encore hémocompatibles et physiologiques.

Les collagènes de type I, II ou III humain ou animal, même après action de la pepsine, possèdent une structure fibrillaire caractéristique en microscopie électronique qui en font de puissants agrégants plaquettaires. Mais comme ces collagènes ne sont pas hémocompatibles, il est préférable d'en éviter l'emploi chaque fois que le médecin utilisateur ne recherche pas l'effet coagulant de contact.

Ces collagènes n'étant pratiquement pas solubles à pH neutre, ils ne permettent que l'obtention de suspensions opaques et il est impossible de les utiliser pour préparer des gels transparents et physiologiques (isotoniques et à pH neutre), sans recourir à une modification chimique des molécules de collagène conduisant à l'apparition de nouveaux déterminants antigéniques. Le brevet américain 3.553.299 THIELE a déjà décrit la fabrication de lentilles de contact à l'aide d'un gel obtenu à partir du cristallin de l'oeil, et donc composé essentiellement de collagène. Les brevets américains 4.223.984 et 4.268.131 MIYATA traitent également de la fabrication de lentilles de contact à partir d'un gel de collagène de type I et qui, pour être soluble à pH neutre, doit être modifié chimiquement.

Les techniques décrites dans ces brevets ne permettent d'obtenir des gels relativement transparents que sur de faibles épaisseurs, et peu résistants mécaniquement. Il n'apparaît par clairement à leur lecture si la transparence est obtenue grâce à une transformation chimique ultérieure (succinylation ou méthylation).

Le brevet américain n° 4.388.428 KUZMA décrit des gels où le collagène est mélangé avec des polymères chimiques pour en améliorer les propriétés mécaniques. Le recours à de tels produits n'est pas souhaitable en raison de la difficulté d'éliminer toute trace de produits toxiques à partir de gels visqueux ou solides.

BRUNS R.R. et GROSS J. (brevet américain n° 4.505.855) confirment la difficulté d'obtenir des gels parfaitement transparents à partir de collagène animal, selon les techniques de l'art antérieur. Ils proposent une méthode d'ultracentrifugation pour réussir une telle préparation, mais cette technique est très onéreuse

3

EP 0 214 035 B1

et difficilement compatible avec une exploitation industrielle.

Par ailleurs, on a déjà prévu d'effectuer des extractions enzymatiques de collagène placentaire par la pepsine, mais les préparations de collagène obtenues ne possèdent pas les propriétés de transparence nécessaires à l'utilisation dans le domaine ophtalmique.

Ainsi M.Z. ABEDIN et R. RIEMSCHNEIDER (Die Angewandte Makromolekulare Chemie, vol. 11, n° 1701, janvier 1983, pages 107-122 Hüthig & Wepf Verlag, Heidelberg) décrivent un procédé de digestion de placenta à la pepsine qui conduit à une contamination du collagène de type IV par les types I et III, nécessitant un fractionnement ultérieur, suivi d'une précipitation et redissolution à des concentrations élevées de NaCl concrétisant un caractère poussé de la digestion.

Ces mêmes auteurs (Die Angewandte Makromolekulare Chemie, vol. 82 n° 1276, 1979, pages 171-186) décrivent un procédé de traitement du placenta de longue durée par l'éther, suivi d'une lyophilisation puis d'un traitement à l'acétate de sodium ou à la soude, avant une digestion à la pepsine, puis d'une dialyse du surnageant pour précipiter le collagène. Celui-ci, après reprise, doit également être précipité à une concentration élevée en NaCl. Ces conditions, qui interdisent d'ailleurs une exploitation à l'échelle industrielle, témoignent également du caractère trop poussé de la digestion et le produit non transparent obtenu paraît être surtout à base de collagène I + III.

Th. F. KRESINA et Ed. J. MILLER (Isolation and characterization of basement membrane collagen from human placental tissue. Evidence for the presence of two genetically distinct collagen chains. Biochemistry, vol. 18, n° 14, 1979, pages 3089-3097, American Chemical Society) décrivent également une digestion pepsinique du tissu placentaire total entraînant une contamination importante par les collagènes de type I et III. Après fractionnement, le collagène doit être traité en pH acide, à des concentrations élevées de NaCl. Le produit obentu n'est pas propre à des utilisations ophtalmiques.

La demande de brevet allemand 2.462.222 décrit un traitement à la pepsine de surnageants d'extractions successives du tissu placentaire et conduit à une solution de collagène trouble.

La demande de brevet européen 0 080 956 décrit une digestion pepsinique d'extraits alcalins du placenta conduisant à du collagène dénaturé et opaque.

La présente invention vise à remédier à ces divers inconvénients en proposant des préparations de collagène, non modifié, non antigénique, parfaitement transparent, physiologique et hémocompatible, et dépourvu de fibres striées observables en microscopie électronique, ces préparations pouvant se présenter notamment sous forme de gel ou de solution.

De façon avantageuse, ces préparations sont très enrichies en type IV ou même contiennent essentiellement du collagène de type IV.

L'invention a pour objet une préparation à usage ophtalmologique ou dermatologique nécessitant la transparence, contenant un collagène essentiellement de type IV, caractérisé en ce que le collagène obtenu par extraction enzymatique du placenta, n'est pas modifié chimiquement, que ledit collagène est dépourvu de fibres striées observables en microscopie électronique, et que ledit collagène donne, dans une solution physiologique, une solution jusqu'à une concentration au plus égale à 2 % et un gel pour une concentration comprise entre 2 % et 30 %.

Les solutions ou gels de collagène obtenus sont transparents sur de grandes épaisseurs pouvant atteindre facilement 20 mm, une épaisseur largement suffisante pour les besoins du marché (lentilles de contact, implants cornéens ou intraoculaires).

Un procédé d'extraction a été mis au point par le demandeur en partant de placentas humains, seule source envisageable pour obtenir cette préparation de collagène humain à l'échelle industrielle.

La connaissance de cette nouvelle technique adaptée au placenta humain a permis au demandeur de l'étendre aux placentas animaux. En effet, les collagènes animaux peuvent être utiles en médecine vétérinaire par exemple ou acceptables pour une utilisation comme dispositif externe non implanté chez l'homme tels que lentilles de contact.

Le procédé conforme à l'invention consiste donc :

- dans un premier temps, à extraire du placenta humain ou animal des collagènes, et à isoler un collagène enrichi en type IV ;
- puis dans un second temps, à préparer, à partir de ce collagène enrichi en type IV, des solutions ou gels présentant les propriétés précédemment décrites.

Le procédé d'extraction des collagènes placentaires conforme à l'invention est caractérisé en ce qu'il comporte les étapes suivantes :

- à partir de placenta humain ou animal, sous forme congelée, que l'on broie ;
- a laver le tissu placentaire broyé, par exemple par une succession de lavages à pH neutre puis à pH acide ;
- à soumettre ledit tissu placentaire lavé à une digestion enzymatique permettant l'extraction du

4

collagène de type IV, pratiquement non contaminé par les collagènes de type I ou III. La digestion s'effectue à l'aide d'une enzyme convenable (à l'exception de la collagénase) et de préférence par la pepsine. Dans ce cas, de préférence, ladite digestion s'effectue à un pH acide, de préférence compris entre 2 et 3,5 à une température, de préférence, comprise entre 0 et 20° C et pendant une durée convenable, notamment de l'ordre de 8 à 24 h, la quantité de pepsine par kg de placenta congelé étant comprise entre 0,5 et 2 g et de préférence entre 0,5 et 1 g ou 1,5 g, soit entre 0,5 et 1 ou 1,5 pour mille.

On peut éventuellement soumettre à une deuxième digestion enzymatique le résidu tissulaire ayant résisté au premier traitement par la pepsine, cette deuxième digestion s'effectuant dans les mêmes conditions que la première digestion et permettant de récupérer ultérieurement, selon des procédés connus, des collagènes de type I et de type III, pratiquement débarrassés de collagène de type IV.

La suspension placentaire ayant subi la première digestion enzymatique est alors traitée par le procédé qui suit, consistant successivement :
- après avoir séparé le tissu résiduel de la suspension placentaire provenant de la digestion enzymatique,
- à effectuer une précipitation à pH acide modéré pour éliminer un précipité d'impuretés,
- à partir du surnageant, à précipiter le collagène par précipitation saline à pH neutre,
- à redissoudre le collagène précipité et neutraliser la solution obtenu,
- à éliminer les impuretés insolubles, et
- à précipiter le collagène par précipitation saline acide.

Ainsi, de façon avantageuse, la suspension placentaire provenant de la digestion enzymatique peut être soumise aux étapes consistant :
- à diluer ladite suspension notamment par un volume d'eau ;
- à ajuster son pH à une valeur comprise entre 7 et 9 ou 10 ;
- à laisser reposer ladite suspension, par exemple pendant 1 à 24 h à une température comprise entre 0 et 20° C ;
- à séparer le tissu résiduel du surnageant de la première digestion ;
- à ajuster le surnageant à un pH acide modéré, par exemple compris entre 4,5 et 5,5 ;
- à laisser reposer ledit surnageant, par exemple pendant 1 à 24 h à une température comprise entre 0 et 20° C ;
- à éliminer la partie insoluble contenant de nombreuses impuretés ;
- à ajuster le surnageant limpide à un pH voisin de la neutralité, de l'ordre de 7,5 ;
- à additioner un sel, tel que par exemple NaCl, jusqu'à l'obtention d'une concentration finale de l'ordre de 1,2 M ;
- à laisser reposer la suspension obtenue pendant par exemple 1 à 24 h à une température comprise entre 10 et 25° C ;
- à récupérer le précipité de collagène formé ;
- à dissoudre le précipité dans une solution faiblement acide ;
- à neutraliser la solution à pH 7,5 ;
- à laisser reposer la suspension obtenue pendant 1 à 24 h à une température comprise entre 0 et 20° C ;
- à éliminer la partie insoluble, éliminant ainsi les impuretés susceptibles de troubler la transparence ;
- à acidifier le surnageant à un pH acide compris par exemple entre 2 et 3,5 ou 4 ;
- à précipiter le collagène par un sel, tel que NaCl, jusqu'à l'obtention d'une concentration finale comprise entre 0,4 et 0,8 M ;
- à laisser reposer la suspension obtenue pendant 1 à 24 h à une température comprise entre 0 et 20° C ;
- et à récupérer le précipité de collagène acide purifié.

Le précipité de collagène purifié obtenu en fin d'opération est repris dans l'acétone ou un solvant organique volatil équivalent, ce qui entraîne la formation de fibres de collagène acide que l'on récupère par filtration sur tamis. Ces fibres sont séchées sous courant d'air tiède et stérile.

Par fibres, on entend ici l'aspect que prend la poudre de collagène solide après séchage, et qui ne correspond nullement à la structure fibreuse striée proprement dite observable en microscopie électronique dans les collagènes connus, en solution.

Dans une variante de réalisation, le précipité de collagène purifié est redissous dans l'eau. Le pH de la solution est ajusté à 7,5 et on lui additionne un sel tel que NaCl, jusqu'à l'obtention d'une concentration finale de l'ordre de 1,2 M. On laisse reposer la suspension obtenue pendant 1 à 24 h et on récupère le précipité de collagène neutre.

Le précipité de fibres de collagène neutre obtenu est repris dans l'acétone ou un solvant organique volatil équivalent, ce qui entraîne la formation de fibres de collagène neutre que l'on récupère par filtration sur tamis. Ces fibres sont séchées sous courant d'air tiède et stérile.

Ce sont ces fibres de collagène acide ou neutre à l'état sec (< 10 % d'eau), qui sont utilisées comme source de collagène pour la préparation de solutions ou gels conformes à l'invention.

La préparation de gels en partant des fibres de collagène acide ou neutre obtenues après le traitement à l'acétone peut s'effectuer comme suit :

- on met les fibres en contact avec un volume d'eau déterminé de façon à obtenir une concentration en collagène comprise entre 2 et 30 % ;
- on laisse gonfler dans l'eau les fibres par exemple pendant environ 1 h ;
- ou soumet l'ensemble à un chauffage modéré ;
- on filtre la solution visqueuse et transparente sur des membranes de porosité comprise entre 0,2 et 8μ;
- on laisse la solution refroidir.

La préparation d'une solution de collagène en partant des fibres de collagène acide enrichi en type IV est réalisée par le procédé suivant :

- on met en contact les fibres de collagène avec un volume d'eau déterminé ;
- on laisse gonfler les fibres de collagène dans l'eau, par exemple pendant environ 1 h ;
- on additionne à l'ensemble une solution d'un sel tel que par exemple l'acétate de sodium, à une concentration inférieure ou égale à 0,1 M pour amorcer la neutralisation du pH.

Les volumes d'eau sont déterminés de façon à obtenir une concentration en collagène au plus égale à 2 %.

- on ajuste la solution à pH 7,5 environ et on la rend éventuellement isotonique par addition de NaCl ;
- on soumet la solution à un chauffage modéré à une température comprise entre 30 et 80°C pendant 10 à 60 minutes. En variante, la solution de collagène peut être filtrée à l'état acide sur des membranes de porosité 0,2 à 8 μ. La neutralisation du pH ne s'effectuant qu'ensuite, dans des conditions de stérilité bactérienne éventuelles, pour assurer la préparation d'un produit stérile, tout en facilitant l'opération de filtration car une solution de collagène est moins visqueuse à pH acide. Une autre variante consiste à filtrer la solution soumise aux ultra-sons pour en réduire momentanément la viscosité ;
- on filtre la solution sur des membranes de porosité comprise entre 0,2 et 8 μ ;
- et on laisse refroidir la solution.

On obtient à l'issue de ces opérations une solution qui présente ces caractéristiques :

- elle est très visqueuse,
- elle contient au plus 2 % de collagène, non dénaturé,
- elle est parfaitement transparente,
- elle est isotonique, physiologique et hémocompatible,
- elle est dépourvue de fibres striées visibles en microscopie électronique.

Une telle solution est susceptible d'être utilisée comme implant en médecine humaine ou animale, dans les domaines de l'ophtalmologie, des articulations osseuses, du traitement des brûlés ou de la dermatologie.

Lorsque l'on utilise comme source de collagène non plus les fibres de collagène acide mais les fibres de collagène neutre enrichi en type IV obtenu selon la variante de réalisation, on applique les mêmes successions d'opérations que celles précédemment décrites pour l'obtention des solutions à partir des fibres de collagène, la neutralisation du pH n'étant plus nécessaire.

Pour préparer une solution de collagène en partant des fibres de collagène neutre enrichi en type IV, on procède comme suit :

- on met en solution les fibres de collagène neutre dans un volume d'eau déterminé de façon à obtenir une concentration en collagène au plus égale à 2 % ;
- on additionne à cette solution un sel tel que NaCl, jusqu'à l'obtention d'une concentration de l'ordre de 0,15 M ;
- on soumet ladite solution à un chauffage modéré à une température comprise entre 30 et 80°C pendant 10 à 60 minutes, et de préférence à une température aux environs de 60°C pendant une dizaine de minutes ;
- on filtre la solution sur des membranes de porosité comprise entre 0,2 et 8 μ ;
- et on laisse refroidir la solution.

On obtient une solution qui présente les mêmes caractéristiques que les solutions de collagène préparées en partant des fibres de collagène acide.

D'autres avantages et particularités de l'invention apparaîtront à la lecture des exemples de réalisation donnés, ci-après, à titre non limitatif.

L'exemple 1 illustre le procédé d'extraction du collagène enrichi en type IV à partir de placentas humains et la préparation de fibres de collagène acide.

L'exemple 2 se rapporte à la préparation de fibres de collagène neutre conforme à l'invention.

L'exemple 3 illustre le procédé d'extraction du collagène enrichi en type IV à partir de placentas animaux.

Les exemples 4 et 5 décrivent la préparation de gels à 15 % de collagène.

Les exemples 6, 7, 8 et 9 décrivent la préparation de solutions collagéniques.

Exemple 1 :

300 kg de placenta sont broyés sous forme congelée pour donner des morceaux de quelques cm3. Le broyat est ensuite mélangé à 300 l d'une solution aqueuse contenant 8 % d'éthanol, 6 g/l de NaCl et 1 kg de cellulose. Après agitation à 10°C, l'ensemble est soumis à une opération de pressage avec un pressoir MABILLE pour séparer le sang du tissu placentaire. On obtient 102 kg de tissu placentaire contenant 65 % d'eau.

Le tissue extrait du pressoir est agité dans 500 l de citrate de sodium à 0,05 M pH 7,2 pendant 30 minutes à 10°C puis soumis au pressage pour éliminer la solution de lavage et récupérer le tissu. Un second lavage est effectué avec 500 l de citrate de sodium à 0,05 M pH 7,2 additionné de 30 g/l de NaCl. Un troisième lavage est effectué avec 500 l de citrate de sodium à 0,05 M pH 7,2. Le tissu ainsi lavé à pH neutre est ensuite soumis à trois séquences successives de lavage à pH acide et à 10°C :
- par 500 l d'acide citrique 0,05 M et à pH final ajusté à 2,8 par addition de HCl 2N ; l'agitation est de 30 minutes avant l'opération de pressage ;
- par 500 l d'acide formique 0,5 M pendant 15 h ;
- par 500 l d'acide citrique 0,05 M additionné de NaCl 20 g/l pendant 30 mn.

Le tissue placentaire ainsi lavé à pH acide a un aspect blanc qui témoigne d'une bonne élimination des pigments rouges du sang placentaire initial. Le poids obtenu est de 82 kg.

Il est soumis à une digestion enzymatique par la pepsine dans 500 l d'acide 0,05 M pH 2,8 contenant 300 g de pepsine, pendant 15 h à 10°C. La suspension est alors diluée par addition de 500 l d'eau à 10°C. Le pH est ajusté à 7,5 par addition de NaOH 4N, pour dénaturer la pepsine et en supprimer l'action protéasique. Après 15 h d'attente à 10°C, le résidu tissulaire qui contient l'essentiel des collagènes I et III non solubilisés est séparé par une centrifugeuse en continu (Westfalia KG 10006). Le poids de ce résidu est de 103 kg ; il peut être soumis à une deuxième digestion enzymatique identique à la première, suivie de l'extraction et de la séparation de chacun des collagènes I et III selon des procédés décrits dans la littérature.

Le surnageant correspondant à la première digestion enzymatique contient l'essentiel des collagènes et autres macromolécules solubles à pH neutre après l'action de la pepsine. Il contient en particulier du collagène de type IV.

Le pH du surnageant est ajusté à 5 avec HCl 2N et, après 15 h d'attente à 10°C, le précipité formé est éliminé par centrifugation en continu sur centrifugeuse "Bactofuge" Alfa Laval.

Le surnageant recueilli, qui est très limpide, est ajusté à pH 7,5 par addition de NaOH 4N et à une concentration finale de 1,2 M en NaCl. Après 15 h à 16°C, le précipité de collagène formé est récupéré par centrifugation en continu sur centrifugeuse "Bactofuge".

Le précipité de 13 kg est ensuite dissous dans 600 l d'HCl 0,01 N, puis le pH est ajusté à 7,5 par addition de NaOH 4N. Le précipité formé après 15 h d'attente à 4°C est éliminé sur centrifugeuse "Bactofuge" (poids obtenu 10 kg).

Le surnageant limpide est acidifié à pH 2,8 avec HCl 2N et additionné de NaCl jusqu'à une concentration finale de 0,6 M à 4°C.

Après 15 h, le précipité de collagène est recueilli sur centrifugeuse "Bactofuge". Le précipité dont le poids est de 6,5 kg a un aspect fluide. Il est additionné lentement de 7 l d'acétone, ce qui entraîne la formation de fibres de collagène que l'on récupère par filtration sur tamis. Le lavage de ces fibres par plusieurs traitements à l'acétone permet, après séchage sous courant d'air tiède et stérile, d'obtenir 180 g de fibres sèches du produit final. L'analyse de la composition en acides aminés de ces fibres montre une structure proche du collagène de type IV et caractéristique de la famille des collagènes ( ≧30% de glycine, environ 10 % d'acide glutamique, de proline et d'hydroxyproline).

Le tableau ci-après donne les résultats d'analyse de la composition en acides aminés de quatre préparations différentes de collagène humain placentaire réalisées selon le présent exemple (les résultats

7

sont exprimés en nombre de molécules de chaque acide aminé pour 1000 résidus).

| n° lots | 1022 | 1023 | 1025 | 1027 |
|---------|------|------|------|------|
| ASP | 46 | 42 | 41 | 42 |
| HYP | 80 | 91 | 85 | 86 |
| THR | 19 | 18 | 18 | 18 |
| SER | 17 | 15 | 17 | 16 |
| ASN | 28 | 30 | 31 | 25 |
| GLU | 107 | 105 | 105 | 107 |
| GLN | 3 | traces | 1 | traces |
| PRO | 92 | 91 | 96 | 98 |
| GLY | 317 | 327 | 313 | 324 |
| ALA | 41 | 39 | 39 | 43 |
| VAL | 33 | 29 | 34 | 34 |
| CYS | 9 | 6 | 9 | 6 |
| CYSTA | 1 | 1 | 1 | 1 |
| MET | 15 | 26 | 16 | 16 |
| ILE | 36 | 36 | 37 | 36 |
| LEU | 57 | 56 | 57 | 56 |
| TYR | 10 | 7 | 12 | 10 |

| n° lots | 1022 | 1023 | 1025 | 1027 |
|---------|------|------|------|------|
| PHE | 33 | 31 | 39 | 34 |
| ORN | 3 | 1 | 1 | 1 |
| LYS | 9 | 8 | 12 | 12 |
| TRY | 0 | 0 | 0 | 0 |
| HIS | 8 | 8 | 8 | 8 |
| ARG | 32 | 32 | 28 | 27 |
| Total | 999 | 996 | 1000 | 1000 |

Exemple 2 :

Le procédé décrit dans l'exemple 1 a été appliqué de manière rigoureusement identique sur un deuxième lot de 300 kg de placentas humains.

En fin d'opération, le précipité obtenu à pH 2,8 en présence de NaCl 0,6 M à 4° C représentait 6,5 kg. Ce précipité a été redissous dans 200 l d'eau. La solution obtenue a été adjustée à pH 7,5 par addition de NaOH 4N, à + 16° C, et additionnée de NaCl pour obtenir une concentration finale de 1,2 M.

Le précipité formé après 15 h a été récupéré par centrifugation sur centrifugeuse "Bactofuge". Le poids obtenu était de 3450 g. Le précipité, séché à l'acétone comme dans l'exemple 1, a permis d'obtenir 200 g de fibres sèches de collagène neutre, enrichi essentiellement en type IV.

Exemple 3 :

Le procédé décrit dans l'exemple 1 a été appliqué de manière rigoureusement identique sur 180 kg de placentas de vache. Les lavages successifs ont été réalisés avec des volumes de 330 litres. La première digestion à la pepsine a été effectuée par addition de 180 g de pepsine dans 300 l d'acide citrique 0,05 M ajusté à pH 2,8. Les autres étapes de précipitation sélective du collagène étaient identiques à celles de l'exemple 1.

Après séchage final par l'acétone, on a récupéré 130 g de fibres sèches de collagène bovin. L'analyse par électrophorèse en gel de polyacrylamide en présence de sodium dodécyl sulfate (SDS) et avec ou sans agent réducteur (dithiothréitol) permet de démontrer que cette préparation est riche en collagène de type IV.

La dissolution dans de l'eau de ces fibres de collagène bovin donne une solution acide due à la précipitation finale réalisée à pH acide.

Exemple 4 :

1,5 g de fibres sèches de collagène humain préparé selon l'exemple 1 est mis en contact avec 10 ml d'eau distillée. Après gonflement des fibres pendant 1 h, l'ensemble est mis au bain-marie à 70° C pendant 30 minutes. On obtient une solution très visqueuse et transparente qui peut être facilement filtrée sur des membranes de porosité comprise entre 0,8 et 8 μ à cette même température. Après refroidissement, le gel obtenu est incolore, acide et parfaitement transparent comme de l'eau filtrée.

Les contrôles par électrophorèse en polyacrylamide en présence de sodium dodécyl sulfate avec et sans dithiothréitol démontrent la parfaite conservation des molécules de collagène IV pendant l'étape de chauffage.

Ce gel peut être ensuite soumis à des opérations connues de mise en forme de lentille de contact ou de tout autre dispositif à propriétés optiques. Les procédés de moulage et de réticulation par des agents chiniques (aldéhydes) ou des agents physiques (rayonnement γ, β, X ou U.V.) s'appliquent parfaitement.

Exemple 5 :

Les fibres de collagène bovin préparées dans l'exemple 3 sont dissoutes selon le protocole de l'exemple 4. On obtient un gel de propriétés physiques comparables à celles du gel de l'exemple 4.

Exemple 6 :

1 g de fibres de collagène neutre obtenu dans l'exemple 2 est mis en solution dans 90 ml d'eau distillée. La solution finale de pH 7,5 ajustée à 100 ml est additionnée de chlorure de sodium pour obtenir une concentration finale de 0,15 M en tenant compte du chlorure de sodium déjà présent dans le précipité initial.

La solution obtenue contient environ 1 % de protéine, principalement du collagène humain de type IV.

Mise au bain-marie à 60°C pendant 10 minutes, cette solution peut être filtrée sur membranes de porosité comprise entre 0,2 et 8 μ. La filtration peut être facilitée en soumettant la solution à l'action des ultra-sons. Après refroidissement, on obtient ainsi une solution très visqueuse, physiologique, parfaitement transparente et stérile de collagène humain.

Cette solution est hémocompatible. En effet, après dilution dans neuf volumes de sang humain prélevé sur citrate, elle n'entraîne aucune coagulation du sang, aucune agrégation plaquettaire, aucune activation de prothrombine en thrombine.

Exemple 7 :

1 g de fibres de collagène humain préparé selon l'exemple 1 est mis à gonfler pendant 1 h dans 50 ml d'eau et additionné de 50 ml d'acétate de sodium 0,1 M. La solution finale obtenue contient environ 1 % de protéines, principalement du collagène humain de type IV. Elle est ajustée à pH 7,5 et rendue éventuellement isotonique par addition de chlorure de sodium.

Mise au bain-marie à 60°C pendant 10 minutes, cette solution peut être filtrée sur membranes de porosité comprise entre 0,2 et 8 μ. On obtient une solution très visqueuse, physiologique, parfaitement transparente et stérile de collagène humain.

Cette solution est hémocompatible comme la précédente.

Exemple 8 :

Le procédé décrit à l'exemple 7 est répété à l'identique à l'exception de l'adjonction de 50 ml d'acétate de sodium 0,1 M, qui est remplacée par l'adjonction de 50 ml de phosphate de sodium disodique 0,05 M.

La solution obtenue est hémocompatible.

Exemple 9 :

1 g de fibres de collagène préparé selon l'exemple 1 est mis à gonfler pendant 1 h dans 50 ml d'eau. La solution acide obtenue est chauffée 10 mn à 30°C et filtrée sur membranes de porosité 0,2 μ.

Le recours aux ultra-sons peut permettre d'accélérer cette filtration, de même qu'un chauffage jusqu'à 37°C. La solution stérile obtenue est ensuite additionnée de 50 ml d'acétate de sodium 0,1 M. Le pH est ajusté à 7,5 avec addition éventuelle de chlorure de sodium pour obtenir un soluté physiologique. L'ensemble des opérations après la filtration peut s'effectuer à l'abri des contaminations bactériennes.

On obtient une solution identique aux précédentes.

**Revendications**

1. Préparation à usage ophtalmologique ou dermatologique nécessitant la transparence, contenant un collagène essentiellement de type IV, caractérisé en ce que le collagène, obtenu par extraction enzymatique du placenta, n'est pas modifié chimiquement, en ce que ledit collagène est dépourvu de fibres striées observables en microscopie électronique et en ce que ledit collagène donne, dans une solution physiologique, une solution jusqu'à une concentration au plus égale à 2 % et un gel pour une concentration comprise entre 2 % et 30 %.

2. Solution ou gel de collagène selon la revendication 1, caractérisé en ce que le collagène résiste à la dénaturation lorsqu'il subit un chauffage modéré, à une température comprise entre 30 et 80°C pendant 10 à 60 minutes, et ce que le critère d'analyse repose sur l'absence de modification du poids moléculaire des différents constituants, par électrophorèse en gel de polyacrylamide avec sodium dodécyl sulfate et avec ou sans agent réducteur.

3. Procédé d'extraction des collagènes placentaires, pour l'obtention d'une préparation selon l'une des revendications 1 et 2 dans lequel on effectue une digestion enzymatique de placenta, notamment à la pepsine, caractérisé en ce que, à partir de placenta humain ou animal, sous forme congelée, que l'on broie et lave,
   - on soumet le tissu placentaire lavé à une digestion enzymatique, ladite digestion étant effectuée pour la pepsine à un pH acide, notamment compris entre 2 et 3,5 à une température convenable, notamment comprise entre 0° et 20°C pendant une durée convenable, notamment comprise entre 8 et 24 h, la teneur en pepsine par rapport au placenta congelé étant comprise entre 0,5 et 1,5 pour 1000, et, après avoir séparé le tissu résiduel de la suspension provenant de la digestion enzymatique, on effectue une précipitation à pH acide modéré pour éliminer un précipité d'impuretés, on recueille le surnageant dont on précipite le collagène par précipitation saline à pH neutre, on redissout le collagène précipité en solution faiblement acide que l'on neutralise, on élimine les impuretés insolubles, et on précipite le collagène par précipitation saline acide.

4. Procédé selon la revendication 3, caractérisé en ce que l'on soumet à une deuxième digestion enzymatique le résidu tissulaire ayant résisté au premier traitement par la pepsine, cette deuxième digestion s'effectuant dans les mêmes conditions que la première digestion et permettant de récupérer ultérieurement des collagènes de type I et de type III, pratiquement débarrassés de collagène de type IV.

5. Procédé selon l'une quelconque des revendications 3 et 4, caractérisé en ce qu'après la digestion à la pepsine
   - on dilue ladite suspension par un volume d'eau,
   - on ajuste son pH à une valeur comprise entre 7 et 10, et de préférence, à une valeur de l'ordre de 7,5,
   - on laisse reposer ladite suspension pendant 1 à 24 h à une température comprise entre 0 et 20°C,
   - on sépare le tissu résiduaire du surnageant, le tissu résiduaire contenant l'essentiel des collagènes de type I et III, tandis que le surnageant contient le collagène de type IV pratiquement non contaminé par les collagènes de type I et III.

6. Procédé selon l'une quelconque des revendications 3 à 4, caractérisé en ce que, pour la précipitation à pH acide modéré,
   - on sépare le tissu résiduel du surnageant de la première digestion,
   - on ajuste le surnageant à un pH compris entre 4,5 et 5,5,
   - on laisse reposer ledit surnageant pendant 1 à 24 h à une température comprise entre 0 et 20°C, et
   - on élimine la partie insoluble.

7. Procédé selon l'une quelconque des revendications 3 à 6, caractérisé en ce que, pour la précipitation saline à pH neutre,
   - on ajuste le surnageant à un ph de 7,5,
   - on additionne un sel, tel que Nacl, jusqu'à l'obtention d'une concentration de 1,2 M,
   - on laisse reposer ledit surnageant pendant 1 à 24 h et à température comprise entre 10 et 25°C,
   - on récupère le précipité de collagène formé.

8. Procédé selon l'une quelconque des revendications 3 à 7, caractérisé en ce que, pour l'élimination des impuretés,
   - on dissout le précipité dans une solution faiblement acide,
   - on neutralise la solution à pH 7,5,
   - on laisse reposer ladite solution pendant 1 à 24 h à une température comprise entre 0 et 20°C,
   - on élimine la partie insoluble.

9. Procédé selon l'une quelconque des revendications 3 à 8, caractérisé en ce que pour précipiter le collagène purifié,
   - on acidifie le surnageant à un pH compris entre 2 et 3,5 à 4,
   - on précipite le collagène par un sel, tel que NaCl, jusqu'à l'obtention d'une concentration finale comprise entre 0,4 M et 0,8 M,
   - on laisse reposer la solution pendant 1 à 24 h à une température comprise entre 0 et 20°C,
   - on récupère le précipité de collagène purifié.

10. Procédé selon l'une quelconque des revendications 3 à 9, caractérisé en ce qu'après avoir redissous le précipité de collagène purifié,
   - on effectue une précipitation saline à pH neutre du collagène.

11. Procédé selon la revendication 10, caractérisé en ce que :
   - l'on redissout dans l'eau le précipité final de collagène acide purifié,
   - l'on ajuste à pH neutre la solution,
   - l'on additionne à la solution un sel, tel que NaCl, jusqu'à l'obtention d'une concentration de 1,2 M,
   - l'on laisse reposer la solution pendant 1 à 24 h, et
   - l'on récupère le précipité de collagène formé, ledit précipité étant utilisé comme source de collagène purifié pour la préparation des solutions ou gels par remise en contact avec une solution aqueuse.

12. Procédé selon l'une quelconque des revendications 5 à 11, caractérisé en ce que :
   - l'on reprend le précipité de collagène purifié acide ou neutre dans un solvant organique volatil, notamment l'acétone, ce qui conduit à la formation de fibres de collagène,
   - l'on récupère et l'on sèche lesdites fibres, celles-ci étant utilisées comme source de collagène purifié pour la préparation des solutions ou gels par remise en contact avec une solution aqueuse.

13. Procédé selon la revendication 12, caractérisé en ce que des gels de collagène filtrés sont obtenus :
   - en mettant en contact les fibres de collagène avec un volume d'eau déterminé de façon à obtenir une concentration en collagène comprise entre 2 et 30 %,
   - en laissant gonfler lesdites fibres, notamment pendant environ 1 h,
   - en soumettant lesdites fibres à un chauffage modéré, notamment à une température comprise entre 30 et 80°C pendant 10 à 60 minutes, et de préférence aux environs de 70°C pendant environ trente minutes,
   - en filtrant la solution transparente et visqueuse obtenue sur une membrane de porosité comprise entre 0,8 et 8µ, - et en laissant refroidir ladite solution.

14. Procédé selon la revendication 12, prise en combinaison avec l'une quelconque des revendications 5 à 9, caractérisé en ce que des solutions de collagène filtrées, sont obtenues :
   - en mettant en contact les fibres de collagène avec un volume d'eau déterminé,
   - en laissant gonfler lesdites fibres dans l'eau pendant notamment environ 1 h,
   - en additionnant à l'ensemble une solution d'un sel tel que l'acétate de sodium, à une concentration de l'ordre de 0,1 M, les volumes d'eau étant déterminés de façon à obtenir une concentration en collagène au plus égale à 2 %,
   - en ajustant le pH à environ 7,5,
   - en soumettant ladite solution à un chauffage modéré, notamment à une température comprise entre 30 et 80°C pendant 10 à 60 minutes,
   - en la filtrant sur des membranes de porosité comprise entre 0,2 et 8 µ,
   - puis en refroidissant ladite solution.

**15.** Procédé selon la revendication 12, prise en combinaison avec l'une des revendications 10 et 11, caractérisé en ce que des solutions de collagène filtrées sont obtenues :
- en mettant en solution le précipité de collagène dans un volume d'eau déterminé de façon à obtenir une concentration en collagène au plus égale à 2 %,
- en additionnant à la solution un sel tel que NaCl, jusqu'à l'obtention d'une concentration de l'ordre de 0,15 M,
- en soumettant ladite solution à un chauffage modéré, notamment à une température comprise entre 30 et 80° C pendant 10 à 60 minutes,
- en filtrant la solution sur des membranes de porosité comprise entre 0,2 et 8μ,
- en laissant refroidir ladite solution.

**16.** Procédé selon l'une des revendications 13 à 15, caractérisé en ce que l'on soumet la solution à l'action d'ultra-sons pendant la filtration.

## Claims

**1.** Preparation for ophthalmological or dermatological use for which transparency is required, containing a collagen essentially of type IV, characterized in that the collagen obtained by enzymatic extraction from placenta is not modified chemically, in that said collagen is devoid from striated fibres observable under an electron microscope and in that said collagen gives, in a physiological solution, either a solution up to a collagen concentration at the most equal to 2 % or a gel with a collagen concentration of between 2 % and 30 %.

**2.** Solution or gel of collagen according to claim 1, characterized in that the collagen resists denaturation when subjected to moderate heating at a temperature of between 30 and 80° C for 10 to 60 minutes, and in that the criterion in the analysis by polyacrylamide gel electrophoresis with sodium dodecyl sulfate with or without a reducing agent is based on the absence of any change in the molecular weight of the various constituents.

**3.** A method for the extraction of placental collagens to obtain a preparation according to one of claims 1 and 2, in which there is carried out an enzymatic digestion of placenta, in particular with pepsin, characterized in that starting with human or animal placenta in deep-frozen form which is ground and washed,
- the washed placental tissue is subjected to enzymatic digestion, said digestion being carried out for pepsin at an acid pH, in particular between 2 and 3.5 at a suitable temperature, in particular between 0° and 20° C for a suitable time, in particular between 8 and 24 h, the pepsin content with respect to the deep-frozen placenta being between 0.5 and 1.5 per 1000, and, after having separated the residual tissue from the suspension derived from the enzymatic digestion, precipitation is made at a moderate acid pH to remove a precipitate of impurities, the supernatant layer is collected and the collagen it contains precipitated by precipitation with a salt at a neutral pH, the precipitated collagen is redissolved in a weakly acid solution which is neutralized, the insoluble impurities are removed and the collagen precipitated by acid precipitation with a salt.

**4.** Method according to claim 3, characterized in that the tissue residue having resisted the first treatment with pepsin is subjected to a second enzymatic digestion, this second digestion being carried out under the same conditions as the first digestion and enabling type I and type III collagens practically free from type IV collagen to be recovered subsequently.

**5.** Method according to any one of claims 3 and 4, characterized in that after pepsin digestion,
- said suspension is diluted with a volume of water,
- its pH is adjusted to a value of between 7 and 10, and preferably at a value of the order of 7.5,
- said suspension is allowed to rest for 1 to 24 h at a temperature of between 0 and 20° C,
- the residual tissue is separated from the supernatant layer, the residual tissue containing the essential of the type I and III collagens, whereas the supernatant layer contains the type IV collagen practically uncontaminated by type I and III collagens.

**6.** Method according to any one of claims 3 to 4, characterized in that for the precipitation at a moderate acid pH,

- the residual tissue is separated from the supernatant layer of the first digestion,
- the pH of the supernatant layer is adjusted between 4.5 and 5.5,
- said supernatant layer is allowed to rest for 1 to 24 h at a temperature of between 0 and 20°C, and
- the insoluble part is removed.

7. Method according to any one of claims 3 to 6, characterized in that for precipitation with a salt at a neutral pH,
   - the pH of the supernatant layer is adjusted to 7.5,
   - a salt, such as NaCl, is added until a concentration of 1.2 M is obtained,
   - said supernatant layer is allowed to rest for 1 to 24 h at a temperature of between 10 and 25°C,
   - the collagen precipitate formed is recovered.

8. Method according to any one of claims 3 to 7, characterized in that in order to remove the impurities,
   - the precipitate is dissolved in a weakly acid solution,
   - the solution is neutralized to a pH of 7.5,
   - said solution is allowed to rest for 1 to 24 h at a temperature of between 0 and 20°C,
   - the insoluble part is removed.

9. Method according to any one of claims 3 to 8, characterized in that to precipitate the purified collagen,
   - the supernatant layer is acidified to a pH of between 2 and 3.5 to 4,
   - the collagen is precipitated with a salt, such as NaCl, until a final concentration of between 0.4 M and 0.8 M is obtained,
   - the solution is allowed to rest for 1 to 24 h at a temperature of between 0 and 20°C,
   - the purified collagen precipitate is recovered.

10. Method according to any one of claims 3 to 9, characterized in that after having redissolved the precipitate of purified collagen,
    - the collagen is precipitated with a salt at a neutral pH.

11. Method according to claim 10, characterized in that :
    - the final precipitate of purified acid collagen is redissolved in water,
    - - the pH of the solution is adjusted to neutral,
    - a salt, such as NaCl, is added to the solution until a concentration of 1.2 M is obtained,
    - the solution is allowed to rest for 1 to 24 h, and
    - the collagen precipitate formed is recovered, said precipitate being used as a source of purified collagen for the preparation of solutions or gels, by being brought again into contact with an aqueous solution.

12. Method according to any one of claims 5 to 11, characterized in that :
    - the acid or neutral purified collagen precipitate is taken up with a volatile, organic solvent, in particular acetone, which results in the formation of collagen fibres,
    - said fibres are recovered and dried, said fibers being used as a source of purified collagen for the preparation of solutions or gels by being brought again into contact with an aqueous solution.

13. Method according to claim 12, characterized in that filtered collagen gels are obtained :
    - by bringing the collagen fibres into contact with a volume of water determined so as to obtain a collagen concentration of between 2 and 30 %,
    - by allowing said fibres to swell, in particular for about 1 h,
    - by subjecting said fibres to moderate heating, in particular at a temperature of between 30 and 80°C for 10 to 60 minutes and preferably of about 70°C for about thirty minutes,
    - by filtering the transparent, viscous solution obtained through a membrane of pore size between 0.8 and 8 μ,
    - and allowing said solution to cool.

14. Method according to claim 12, in combination with any one of claims 5 to 9, characterized in that filtered collagen solutions are obtained :
    - by bringing the collagen fibres into contact with a determined volume of water,

- by allowing said fibres to swell in the water, in particular for about 1 h,
- by adding to the whole a solution of a salt, such as sodium acetate, at a concentration of the order of 0.1 M, the volumes of water being determined so as to obtain a collagen concentration at the most equal to 2 %,
- by adjusting the pH to about 7.5,
- by subjecting said solution to moderate heating, in particular at a temperature of between 30 and 80° C for 10 to 60 minutes,
- by filtering said solution through membranes of pore size between 0.2 and 8 $\mu$,
- then cooling said solution.

15. Method according to claim 12, in combination with any one of claims 10 and 11, characterized in that the filtered collagen solutions are obtained :
- by dissolving the collagen precipitate in a volume of water determined so as to obtain a collagen concentration at the most equal to 2%,
- by adding a salt, such as NaCl, to the solution until a concentration of the order of 0.15 M is obtained,
- by subjecting said solution to moderate heating, in particular at a temperature of between 30 and 80° C for 10 to 60 minutes,
- by filtering the solution through membranes of pore size between 0.2 and 8 $\mu$,
- then allowing said solution to cool.

16. Method according to one of claims 13 to 15, characterized in that the solution is subjected to the action of ultrasonic waves during filtration.

## Patentansprüche

1. Präparat zur ophtalmologischen oder dermatologischen Anwendung, die Transparenz erfordert, enthaltend ein Kollagen im wesentlichen des Typs IV, **dadurch gekennzeichnet,** daß das durch enzymatische Extraktion der Plazenta erhaltene Kollagen chemisch nicht modifiziert ist und daß das genannte Kollagen von im Elektronenmikroskop feststellbaren Fasern befreit ist und daß das Kollagen in einer physiologischen Lösung eine Lösung bis zu einer Konzentration von höchstens 2% und ein Gel für eine Konzentration zwischen 2% und 30% ergibt,

2. Kollagenlösung oder -gel nach Anspruch 1, dadurch gekennzeichnet, daß das Kollagen der Denaturierung widersteht, wenn es eine mäßige Erwärung auf eine Temperatur zwischen 30° C und 80° C während 10 min bis 60 min erfährt, und das Analysenkriterium auf dem Fehlen der Veränderung des Molekulargewichts der versahiedenen Bestandteile durch Elektrophorese im Gel des Polyacrylamids mit Natriumdodecylsulfat und mit oder ohne Reduktionsmittel beruht.

3. Extraktionsverfahren für Plazentakollagene zur Herstellung eines Präparats nach einem der Ansprüche 1 und 2, bei dem man eine enzymatische Plazentadigerierung, insbesondere auf Pepsin, ausführt, dadurch gekennzeichnet, daß man ausgehend von menschlicher oder tierischer Plazenta in gefrorenem Zustand, die man bricht und wäscht,

das gewaschene Plazentagewebe einer enzymatischen Digerierung unterwirft, die für das Pepsin bei einem sauren pH-Wert, insbesondere zwischen 2 und 3,5, bei einer geeigneten Temperatur, insbesondere zwischen 0° C und 20° C während einer geeigneten Dauer, insbesondere zwischen B h und 24 h, ausgeführt wird, wobei der PepSingehalt in Bezug auf die gefrorene Plazenta zwischen 0,5 und 1,5 0/00 liegt, und nach dem Abtrennen des Restgewebes aus der Suspension, die von der enzymatischen Digerierung stammt, man einen Niederschlag bei saurem, gemäßigtem pH-Wert ausführt, um einen Niederschlag von Verunreinigungen zu beseitigen, man das Aufschwimmende auffängt, von dem man das Kollagen durch salzigen Niederschlag bei neutralem pH-Wert niederschlägt, man das niedergeschlagene Kollegen in schwach saurer Lösung, die man neutralisiert, wieder auflöst, man die unlöslichen Verunreinigungen beseitigt und man das Kollagen durch salzsauren Niederschlag ausfällt.

4. Verfahren nach Anspruch 3, dadurch gekennzeichnet, daß man den Gewebsrückstand, der der ersten Behandlung durch das Pepsin widerstanden hat, einer zweiten enzymatischen Digerierung unterwirft, die sich unter den gleichen Bedingungen wie die erste Digerierung vollzieht und schließlich die Gewinnung der Kollagene des Typs I und des Typs III praktisch vom Kollagen des Typs IV befreit erlaubt.

5. Verfahren nach einem der Ansprüche 3 und 4, dadurch gekennzeichnet, daß nach der Digerierung auf das Pepsin man

die Suspension mit einer Wassermenge verdünnt,

ihr pH auf einen Wert einstellt, der zwischen 7 und 10 liegt und vorzugsweise einen Wert in der Größenordnung von 7,5 hat,

die genannte Suspension während 1 h bis 24 h bei einer Temperatur zwischen 0° C und 20° C ruhen läßt,

den Gewebsrückstand vom Aufschwimmenden abtrennt, wobei der Gewebsrückstand im wesentlichen Kollagene Vom Typ I und Typ III enthält, während das Aufschwimmende das Kollagen von Typ IV enthält, das durch die collage vom Typ I und III praktisch nicht kontaminiert ist.

6. Verfahren nach einem der Ansprüche 3 und 4, dadurch gekennzeichnet, daß für die Abscheidung bei mäßig saurem pH man

die Gewebsrückstände vom Aufschwimmenden der ersten Digerierung abtrennt,

das Aufschwimmende auf einen pH zwischen 4,5 und 5,5 einstellt,

das Aufschimmende während 1 h bis 24 h bei einer Temperatur zwischen 0° C und 20° C ruhen läßt, und

den unlöslichen Anteil beseitigt.

7. Verfahren nach einem der Ansprüche 3 bis 6, dadurch gekennzeichnet, daß für die salzige Abscheidung bei neutralem pH man

das Aufschwimmende auf ein pH von 7,5 einstellt,

ein Salz, wie beispielsweise Kochsalz, hinzufügt, bis eine Konzentration von 1,2 M erzielt ist,

das Aufschwimmende während 1 h bis 24 h und bei einer Temperatur zwischen 10° C und 23° C ruhen läßt,

den gebildeten Kollagenniederschlag abzieht.

8. Verfahren nach einem der Ansprüche 3 bis 7, dadurch gekennzeichnet, daß für die Beseitigung von Verunreinigungen,

man den Niederschlag in einer schwach sauren Lösung auflöst,

man die Lösung auf pH 7,5 neutralisiert,

man die Lösung während 1 h bis 24 h bei einer Temperatur zwischen 0° C und 20° C ruhen läßt,

man den unlöslichen Anteil beseitigt.

9. Verfahren nach einem der Ansprüche 3 bis 8, dadurch gekennzeichnet, daß für die Abscheidung des gereinigten Kollagens man das Aufschwimmende auf ein pH zwischen 2 und 3,5 bis 4 ansäuert,

man das Kollagen durch ein Salz, wie beispielsweise Kochsalz, ausfällt, bis man eine Endkonzentration zwischen 0,4 M und 0,8 M erzielt,

man die Lösung während 1 h bis 24 h bei einer Temperatur zwischen 0°C und 20°C ruhen läßt,

man den gereinigten Kollagenniederschlag abzieht.

10. Verfahren nach einem der Ansprüche 3 bis 9, dadurch gekennzeichnet, daß man nach der Wiederauflösung des gereinigten Kollagen niederschlags

man einen salzigen Niederschlag des Kollagens bei neutralem pH ausführt.

11. Verfahren nach Anspruch 10, dadurch gekennzeichnet, daß man den Endniederschlag des gereinigten sauren Kollagens in Wasser wieder auflöst,

man die Lösung auf ein neutrales pH einstellt,

man zu der Lösung ein Salz, beispielsweise Kochsalz, hinzufügt, bis eine Konzentration von 1,2 M erreicht ist,

man die Lösung während 1 h bis 24 h ruhen läßt, und

man den gebildeten Kollagenniederschlag abzieht, der als Ausgangsmaterial für gereinigtes Kollagen für die Zubereitung von Lösungen oder Gels durch Inberührungbringen mit einer wässrigen Lösung verwendet wird.

12. Verfahren nach einem der Ansprüche 5 bis 11, dadurch gekennzeichnet, daß

man den gereinigten sauren oder neutralen Kollagenniederschlag wieder in einem flüchtigen organischen Lösungsmittel, insbesondere Aceton, aufnimmt, was zur Bildung von Kollagenfasern führt,

man die genannten Fasern abzieht und trocknet, die als Ausgangsmaterial für gereinigtes Kollagen für die Zubereitungen von Lösungen oder Gels durch Inberührungbringen mit einer wässrigen Lösung verwendet werden.

13. Verfahren nach Anspruch 12, dadurch gekennzeichnet, daß man gefilterte Kollagengels erhält,

indem man die Kollagenfasern mit einer vorbestimmten Wassermenge in Berührung bringt, um eine Kollagenkonzetration zwischen 2% und 30% zu erhalten,

man die genannten Fasern insbesondere während etwa 1 h aufquellen läßt,

man die Fasern einer mäßigen Erwärmung, insbesondere bei einer Temperatur zwischen 30°C und 80°C während 10 min bis 60 min und vorzugsweise bei etwa 70°C während etwa 30 min unterwirft,

man die erhaltene transparente und viskose Lösung über eine Membran einer Porosität zwischen 0,8 und 8 μ filtriert,

und man die Lösung wieder abkühlen läßt.

14. Verfahren nach Anspruch 12 in Kombination mit einem der Ansprüche 5 bis 9, dadurch gekennzeichnet, daß die gefilterten Kollagenlösungen erhalten werden, indem:

man die Kollagenfasern mit einer vorbestimmten wassermenge in Berührung bringt,

man die genannten Fasern in dem Wasser während insbesondere etwa 1 h aufquellen läßt,

man dem Gemisch eine Lösung eines Salzes, wie Natriumacetat, einer Konzentration in der Größenordnung von 0,1 M hinzufügt, wobei die Wassermengen derart bestimmt sind, daß man eine Kollagenkonzentration von höchstens gleich 2% erhält,

17

man das pH auf etwa 7,5 einstellt,

man die Lösung einer mäßigen Erwärmung insbesondere bei einer Temperatur zwischen 30°C und 80°C während 10 min bis 60 min unterwirft,

man sie über Membranen einer Porosität zwischen und 0,2 μ und 8 μ filtriert,

man dann die Lösung wieder abkühlt.

15. Verfahren nach Anspruch 12 in Kombination mit einem der Ansprüche 10 und 11, dadurch gekennzeichnet, daß man filtrierte Kollagenlösungen erhält, indem:

man den Kollagenniederschlag in einer vorbestimmten Wassermenge in Lösung bringt, daß man eine Kollagenkonzentration von höchstens 2% erhält,

man zu der Lösung ein salz, wie beispielsweise Kochsalz, hinzufügt, bis eine Konzentration in der Größenordnung von 0,15 M erzielt ist,

man die Lösung einer mäßigen Erwärmung, insbesondere bei einer Temperatur zwischen 30°C und 80°C während 10 min bis 60 min unterwirft,

man die Lösung über Membranen einer Porosität zwischen 0,2 μ und 8 μ filtriert,

man die Lösung abkühlen läßt.

16. Verfahren nach einem der Ansprüche 13 bis 15, dadurch gekennzeichnet, daß man die Lösung während der Filtrierung der Einwirkung von Ultraschall aussetzt.